# EUROPÄISCHE PATENTSCHRIFT

(11) **EP 1 571 164 B1**
(45) Veröffentlichungstag und Bekanntmachung des Hinweises auf die Patenterteilung: **10.09.2008**
(21) Anmeldenummer: 05003617.7
(22) Anmeldetag: 19.02.2005
(51) Int. Cl.: C08F 220/18, C08F 2/26, A61K 9/20, A61K 9/50

(54) **Verwendung wässriger Polymerdispersionen auf Basis von Alkyl(meth)-aycrylaten**
Use of aqueous polymer dispersions based on alkyl (meth)acrylates
Utilisation des dispersions de polymères aqueuses à base de alkyl (meth)acrylates

(30) Priorität: 05.03.2004 DE 102004011349
(43) Veröffentlichungstag der Anmeldung: 07.09.2005
(73) Patentinhaber: BASF SE, 67056 Ludwigshafen (DE)
(72) Erfinder: Kolter, Karl Dr., 67117 Limburgerhof (DE); Angel, Maximilian, Dr., 67105 Schifferstadt (DE)

(56) Entgegenhaltungen:
- EP-A- 0 590 604
- DE-A1- 2 135 073
- US-A- 4 937 282

## Beschreibung

Die Erfindung betrifft die Verwendung wässriger Polymerdispersionen mit einem verminderten Emulgatorgehalt als Binde- und Überzugsmittel für pharmazeutische Darreichungsformen.

Die Tatsache, dass Stabilisatoren in pharmazeutischen Dispersionen die Filmbildung negativ beeinflussen, ist lange bekannt. So wird in "Aqueous Polymeric coatings for Pharmaceutical Dosage Forms", 2nd Edition, Ed. James W. McGinity 1997, Marcel Dekker Inc., New York, Seiten 56-67, ausgeführt, dass Surfactants und wasserlösliche Additive sehr nachteilige Wirkungen auf die Verfilmung und die Filmeigenschaften haben. Insbesondere wird Natriumlaurylsulfat erwähnt, das die Struktur, die mechanischen und Permeationseigenschaften von Filmen verändert, weil es während der Filmbildung in kleine Zwischenräume zwischen den Polymerteilchen wandert. Außerdem wird Natriumlaurylsulfat für die unerwünschten Eigenschaften der pH-Abhängigkeit der Freisetzung und für die Veränderung bei Lagerung verantwortlich gemacht. Auch hydrophile Verbindungen wie Polyethoxylate können sehr nachteilige Auswirkungen auf die Filme haben. So kommt es beispielsweise zu Phasenseparation, Flocculation, Beschleunigung der Wirkstofffreisetzung, Verschlechterung der mechanischen Eigenschaften und ähnlichen unerwünschten Phänomenen.

Aus der WO 03/087180 ist bekannt, dass solche Probleme durch Verwendung von Überzugsdispersionen, die gar keine Hilfsstoffe wie Emulgatoren mehr enthalten, vermieden werden soll. Statt dessen werden Verbindungen mit Emulgatoreigenschaften, wie beispielsweise Ester der (Meth-)Acrylsäure mit Polyethylenglykol, in die filmbildenden Polymere einpolymerisiert.

Aus der DE 100 11 447 ist bekannt, dass Dispersionen, die Nonylphenolethoxilate als Emulgator enthalten, Nachteile aufweisen. Gemäß diesem Stand der Technik soll das Problem der Stabilisierung von Dispersionen von Methacrylat-copolymeren durch Verwendung von nichtionischen Emulgatoren mit einem HLB-Wert von 15,7 bis 16,2 gelöst werden. Allerdings weisen die bevorzugten Dispersionen noch einen relativ hohen Emulgatorgehalt auf.

Aufgabe der vorliegenden Erfindung war es, Dispersionen zu finden, die einen möglichst geringen Emulgatoranteil aufweisen, aber gleichzeitig gute anwendungstechnische Eigenschaften als Binde- und Überzugsmittel in pharmazeutischen Darreichungsformen haben.

Die Aufgabe wurde erfindungsgemäß gelöst durch wässrige Polymerdispersionen, erhalten durch radikalische Polymerisation in einem wässrigen System in Gegenwart eines Polymerisationsinitiators von
i) 60 bis 99,95 Gew.-% mindestens eines Monomers ausgewählt aus der Gruppe bestehend aus Alkylacrylaten, Alkylmethacrylaten , und Vinylestern C₁-C₂₄-Carbonsäuren,
ii) 0,05 bis 5 Gew.-% einer monolefinisch ungesättigten C3-C8-Carbonsäure
iii) 0 bis 35 Gew.-% weiterer radikalisch polymerisierbarer Monomere,
wobei die Summe von i), ii), und iii) gleich 100 Gew.-% ist, in Gegenwart von 0,001 bis 1,0 Gew.-%, bezogen auf das Gesamtgewicht der Monomeren, eines anionischen Emulgators.

Bevorzugte Polymerisate werden erhalten aus
i) 95 bis 99.95 Gew.-% mindestens eines Monomers ausgewählt aus der Gruppe bestehend aus Alkylacrylaten, Alkylmethacrylaten und Vinylestern von C₁-C₂₄-Carbonsäuren,
ii) 0,05 bis 5 Gew.-% einer monolefinisch ungesättigten C3-C8-Carbonsäure.

Als erfindungsgemäß geeignete, radikalisch polymerisierbaren Monomeren i) eignen sich Verbindungen aus der Gruppe, bestehend aus C₁-C₂₄-Alkylestern von Acrylsäure und von Methacrylsäure, und C₁-C₂₄-Alkylvinylestern.

Als Alkylester von Acrylsäure und Methacrylsäure seien verzweigte oder unverzweigte C₁-C₂₄-Alkylester, bevorzugt Methyl-, Ethyl-, n-Propyl-, 1-Methylethyl-, n-Butyl-, 1-Methylpropyl-, 2-Methylpropyl-, 1,1-Dimethylethyl-, n-Pentyl-, 1-Methylbutyl-, 2-Methylbutyl-, 3-Methylbutyl-, 2,2-Dimethylpropyl-, 1-Ethylpropyl-, n-Hexyl-, 1,1-Dimethylpropyl-, 1,2-Dimethylpropyl-, 1-Methylpentyl-, 2-Methylpentyl-, 3-Methylpentyl-, 4-Methylpentyl-, 1,1-Dimethylbutyl-, 1,2-Dimethylbutyl-, 1,3-Dimethylbutyl-, 2,2-Dimethylbutyl-, 2,3-Dimethylbutyl-, 3,3-Dimethylbutyl-, 1-Ethylbutyl-, 2-Ethylbutyl-, 1,1,2-Trimethylpropyl-, 1,2,2-Trimethylpropyl-, 1-Ethyl-1-methylpropyl-, 1-Ethyl-2-methylpropyl-, n-Heptyl-, 2-Ethylhexyl-, n-Octyl-, n-Nonyl-, n-Decyl-, n-Undecyl-, n-Dodecyl-, n-Tridecyl-, n-Tetradecyl-, n-Pentadecyl-, n-Hexadecyl-, n-Heptadecyl-, n-Octadecyl-, n-Nonadecyl-, n-Eicosyl-, n-Docosyl- oder n-Tetracosylester genannt.

Als bevorzugte Vertreter der oben genannten Alkylreste seien verzweigte oder unverzweigte C₁-C₁₂-, besonders bevorzugt C₁-C₆-Alkylreste genannt. Insbesondere eignen sich Ethylacrylat und Methylmethacrylat.

Eine bevorzugte Ausführungsform der Erfindung betrifft Polymerisate die als Monomere i) C₁-C₆-Alkylester von Acrylsäure und/oder Methacrylsäure enthalten.

Eine weitere bevorzugte Ausführungsform betrifft Polymerisate die jeweils ein Alkylacrylat und ein Alkylmethacrylat enthalten.

Weiterhin kommen als Monomere i) Vinylester in Betracht. Als Vinylester von aliphatischen C₁-C₂₄-Carbonsäuren eignen sich Vinylester von aliphatischen verzweigten oder unverzweigten, gesättigten oder ungesättigten C₁-C₂₄-Carbonsäuren. Beispielsweise seien genannt Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure sowie Lignocerinsäure.

Bevorzugt werden Vinylester der oben genannten C₁-C₁₂-Carbonsäuren, insbesondere der C₁-C₆-Carbonsäuren verwendet. Ganz besonders bevorzugt ist Vinylacetat.

Als Monomere ii) eignen sich monoethylenisch ungesättigte Carbonsäuren mit 3 bis 8 C-Atomen, beispielsweise Acrylsäure, Methacrylsäure, Dimethacrylsäure, Ethacrylsäure, Allylessigsäure, Vinylessigsäure, Crotonsäure und Maleinsäure. Bevorzugte Monomere ii) sind Acrylsäure und Methacrylsäure.

Als weitere Monomere iii) eignen sich Amide von monoethylenisch ungesättigte Carbonsäuren mit 3 bis 8 C-Atomen wie beispielsweise Amide von Acrylsäure, Methacrylsäure, Dimethacrylsäure, Ethacrylsäure, Allylessigsäure, Vinylessigsäure oder Crotonsäure, bevorzugt aus dieser Gruppe von Carbonsäuren sind Acrylsäure und/oder Methacrylsäure.
Als bevorzugte Vertreter dieser Gruppe seien Acrylamid (H₂C=CH-CO-NH₂) und Methacrylamid (CH₂=C(CH₃)-CO-NH₂) genannt.
Als weitere Monomere iii) eignen sich auch C₁-C₂₄-Alkyl-Vinylether, bevorzugt C₁-C₁₂-Alkyl-Vinylether, N-C₁-C₂₄-Alkyl-substituierter Amide von monoethylenisch ungesättigten C₃-C₈-Carbonsäuren, N,N-C₁-C₂₄-Dialkyl-substituierter Amide von monoethylenisch ungesättigten C₃-C₈-Carbonsäuren, N-Vinyllactame, N-Vinylamine, Styrol und Butadien.

Als bevorzugte C₁-C₁₂-Alkylreste der Vinylether seien verzweigte oder unverzweigte Alkylketten wie z.B. Methyl-, Ethyl-, n-Propyl-, 1-Methylethyl-, n-Butyl-, 1-Methylpropyl-, 2-Methylpropyl-, 1,1-Dimethylethyl-, n-Pentyl-, 1-Methylbutyl-, 2-Methylbutyl-, 3-Methylbutyl-, 2,2-Dimethylpropyl-, 1-Ethylpropyl-, n-Hexyl-, 1, 1-Dimethylpropyl-, 1,2-Dimethylpropyl-, 1-Methylpentyl-, 2-Methylpentyl-, 3-Methylpentyl-, 4-Methylpentyl-, 1,1-Dimethylbutyl-, 1,2-Dimethylbutyl-, 1,3-Dimethylbutyl-, 2,2-Dimethylbutyl-, 2,3-Dimethylbutyl-, 3,3-Dimethylbutyl-, 1-Ethylbutyl-, 2-Ethylbutyl-, 1,1,2-Trimethylpropyl-, 1,2,2-Trimethylpropyl-, 1-Ethyl-1-methylpropyl-, 1-Ethyl-2-methylpropyl-, n-Heptyl-, 2-Ethylhexyl-, n-Octyl-, 2-Ethylhexyl-, n-Nonyl-, n-Decyl-,n-Undecyl- sowie n-Dodecylreste genannt.

Als weitere Monomere iii) können N-C₁-C₂₄-Alkyl- oder N,N-C₁-C₂₄-Dialkyl-substituierte Amide von monoethylenisch ungesättigten C₃-C₈-Carbonsäuren eingesetzt werden, wobei es sich bei den Alkylresten um verzweigte oder unverzweigte aliphatische Alkylreste mit 1 bis 24 Kohlenstoffatomen handelt, beispielsweise Methyl-, Ethyl-, n-Propyl-, 1-Methylethyl-, n-Butyl-, 1-Methylpropyl-, 2-Methylpropyl-, 1,1-Dimethylethyl-, n-Pentyl-, 1-Methylbutyl-, 2-Methylbutyl-, 3-Methylbutyl-, 2,2-Dimethylpropyl-, 1-Ethylpropyl-, n-Hexyl-, 1,1-Dimethylpropyl-, 1,2-Dimethylpropyl-, 1-Methylpentyl-, 2-Methylpentyl-, 3-Methylpentyl-, 4-Methylpentyl-, 1,1-Dimethylbutyl-, 1,2-Dimethylbutyl-, 1,3-Dimethylbutyl-, 2,2-Dimethylbutyl-, 2,3-Dimethylbutyl-, 3,3-Dimethylbutyl-, 1-Ethylbutyl-, 2-Ethylbutyl-, 1,1,2-Trimethylpropyl-, 1,2,2-Trimethylpropyl-, 1-Ethyl-1-methylpropyl-, 1-Ethyl-2-methylpropyl-, n-Heptyl-, 2-Ethylhexyl-, n-Octyl-, n-Nonyl-, n-Decyl-, n-Undecyl-, n-Dodecyl-, n-Tridecyl-, n-Tetradecyl-, n-Pentadecyl-, n-Hexadecyl-, n-Heptadecyl-, n-Octadecyl-, n-Nonadecyl-, n-Eicosyl-, n-Docosyl- oder n-Tetracosylreste, bevorzugt solche Alkylreste mit 1 bis 12, besonders bevorzugt mit 1 bis 6 Kohlenstoffatomen.

Bevorzugte amidierte Comonomere sind beispielsweise N-Methylacrylamid, N-Ethylacrylamid, N-n-Propylacrylamid, N-1-Methylethylacrylamid, N-n-Butylacrylamid, N-1-Methylpropylacrylamid, N-2-Methylpropylacrylamid, N-1,1-Dimethylethylacrylamid, N-n-Pentylacrylamid, N-n-Hexylacrylamid, N-Methylmethacrylamid, N-Ethylmethacrylamid, N-n-Propylmethacrylamid, N-1-Methylethylmethacrylamid, N-n-Butylmethacrylamid, N-1-Methylpropylmethacrylamid, N-2-Methylpropylmethacrylamid, N-1,1-Dimethylethylmethacrylamid, N-n-Pentylmethacrylamid, N-n-Hexylmethacrylamid.

Als N-Vinyllactame oder N-Vinylamine seien Verbindungen genannt, ausgewählt aus der Gruppe, bestehend aus N-Vinylpyrrolidon, N-Vinylpiperidon, N-Vinylcaprolactam, N-Vinylimidazol, methyliertes N-Vinylimidazol und N-Vinylformamid.

Selbstverständlich können auch Mischungen der jeweiligen oben genannten Monomeren polymerisiert werden.

Der Anteil an Monomeren iii) ist bevorzugt gleich Null.

Ganz besonders bevorzugte Polymerisate werden erhalten aus
i) 95 bis 99.95 Gew.-% Ethylacrylat oder Methylmethacrylat oder Mischungen davon, und
ii) 0,05 bis 5 Gew.-% Methacrylsäure.

Das Gewichtsverhältnis von Alkylacrylaten zu Alkylmethacrylaten beträgt 9:1 bis 1:3, bevorzugt 3:1 bis 1:1, besonders bevorzugt 2,5:1 bis 1,5:1.

Als Emulgatoren verwendet man anionische Verbindungen, bevorzugt anionische Emulgatoren, deren HLB-Wert im Bereich >20 liegt. Der HLB-Wert ist ein von Griffin eingeführtes Mass der Hydrophilie oder Lipophilie und bedeutet Hydrophilic-Lipophilic-Balance. Bei anionischen Emulgatoren kann dieser Wert nur rechnerisch ermittelt werden.

Eine Auflistung der entsprechenden Verbindungen findet sich beispielsweise in Fiedler's Handbuch der pharmazeutischen und kosmetischen Hilfsstoffe, 4. Auflage, 1996, Seiten 82, 84.

Als geeignete anionische Emulgatoren kommen in Betracht:
Ammoniumsalzen oder Alkalisalzen, insbesondere Natrium- und Kaliumsalzen, von C8-C30-Fettsäuren, Alkylsulfaten, Alkylsulfonaten oder Alkylarylsulfonaten, wobei die Alkylreste 8 bis 30, bevorzugt 10 bis 24 C-Atome aufweisen, weiterhin entsprechende Salze von Sulfobemsteinsäure-mono- und diestern oder von sulfatierten Fettalkoholethoxylaten.

Bevorzugte anionische Emulgatoren sind Natriumlaurylsulfat, Natriumdioctylsulfosuccinat, Natriumstearat und Natriumlaurylsulfonat.

Die Emulgatoren werden in Mengen von 0,001 bis 1.0 Gew.-%, insbesondere 0,005 bis 0.95 Gew.-%, bevorzugt 0,01 bis 0,5 Gew.-%, besonders bevorzugt 0,05 bis 0,35 Gew.-%, bezogen auf die Gesamtmenge an eingesetzten Monomeren, eingesetzt.

Zur Herstellung der Polymerisate können die Monomeren in an sich bekannter Weise sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Polymerisationsinitiatoren eignen sich u.a. organische Peroxide und Hydroperoxide, wie Diacetylperoxid, Dibenzoylperoxid, Succinylperoxid, Di-tert.-butylperoxid, tert.-Butylperbenzoat, tert.-Butylperpivalat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid; anorganische Perverbindungen wie Alkaliperoxodisulfate, Ammoniumperoxodisulfat oder H₂O₂; Redoxinitiatoren wie H₂O₂/Ascorbinsäure, H₂O₂/Fe²⁺, Peroxodisulfate/Thiosulfate, Peroxide/Thiosulfate; Azostarter wie 4,4'-Azobisisobutyronitril sowie Mischungen der genannten Initiatoren.

Bevorzugte Vertreter der o.g. Polymerisationsinitiatoren sind Ammoniumperoxodisulfat, die sauer wirkenden Alkaliperoxodisulfate, insbesondere die Natrium- und Kaliumsalze sowie die Redoxinitiatoren H₂O₂/Ascorbinsäure.

Die verwendeten Mengen an Initiator bzw. Initiatorgemischen bezogen auf eingesetztes Monomer liegen zwischen 0,01 und 10 Gew.-%, vorzugsweise zwischen 0,1 und 1,5 Gew.-%, besonders bevorzugt zwischen 0,1 und 0,8 Gew.-%.

Bei Verwendung von überwiegenden Mengen an Vinylestern als Monomere i) kann sich die Zugabe eines Schutzkolloids empfehlen.

Die erfindungsgemäße Polymerisation kann kontinuierlich oder diskontinuierlich durchgeführt werden. Bevorzugt arbeitet man mit dem semi-diskontinuierlichen Verfahren, bei dem man beispielsweise einen Teil Polymerisationsinitiator, Emulgator, Puffersubstanz und Monomer vorlegt, das Gemisch auf Polymerisationstemperatur erhitzt und nach Anspringen der Polymerisation den Rest, d.h. jeweils Polymerisationsinitiator und Monomer gleichzeitig über getrennte Zuläufe zudosiert.

Die Polymerisation erfolgt im Temperaturbereich von 40 bis 200°C, bevorzugt im Bereich von 50 bis 140°C, besonders bevorzugt im Bereich von 60 bis 100°C. Sie wird üblicherweise unter atmosphärischem Druck durchgeführt, kann jedoch auch unter vermindertem oder erhöhtem Druck, vorzugsweise zwischen 1 und 5 bar ablaufen.

Die Reaktionszeiten betragen je nach Ansatzgröße normalerweise 1 bis 10, meistens 1,5 bis 5 Stunden.

Für die Herstellung lagerstabiler, wässriger Polymerdispersionen ist es außerdem von Vorteil, nach vollständiger Umsetzung der polymerisierbaren Monomeren das System auf einen pH-Wert im Bereich von 4 bis 8, bevorzugt im Bereich von 5 bis 7 einzustellen. Die pH-Einstellung kann auf an sich bekannte Weise, beispielsweise durch Zugabe von wässrigen Alkalihydroxid-Lösungen erfolgen.

Der Feststoffgehalt der erhaltenen wässrigen Polymerdispersionen bzw. Lösungen beträgt in der Regel 10 bis 70 Gew.-%, bevorzugt 15 bis 65 Gew.-%, besonders bevorzugt 20 bis 60 Gew.-%.

Das mittlere Molekulargewicht der Polymerisate (Gewichtsmittel) bestimmt durch Lichtstreung beträgt 300.000 und 1.000.000.

Die wässrigen Polymerdispersionen können durch verschiedene Trocknungsverfahren wie z.B. Sprühtrocknung, Fluidized Spray Drying, Walzentrocknung oder Gefriertrocknung in Pulverform überführt werden. Durch die vorteilhafte geringe Viskosität der Polymerdispersionen wird als Trocknungsverfahren bevorzugt die Sprühtrocknung eingesetzt.

Sofern erforderlich können hierbei auch Sprühhilfsmittel wie z.B. hochdisperse Kieselsäure, Silikate, Stärke bzw. Stärkederivate, Cellulose bzw. Cellulosederivate, Polyvinylpyrrolidone oder Polyvinylalkohole zugesetzt werden.

Aus dem so erhaltenen Polymer-Trockenpulver läßt sich durch Redispergieren in Wasser erneut eine wässrige Dispersion bzw. Lösung herstellen. Die Überführung in Pulverform hat den Vorteil einer einfacheren Transportmöglichkeit sowie einer geringeren Neigung für Keimbefall.

Die Polymerisate eignen sich hervorragend als Retardierungspolymere zur Verzögerung der Wirkstofffreisetzung.

Ein bevorzugtes Einsatzgebiet der wasserdispergierbaren Polymerisate ist die Verwendung als Überzugsmittel für feste pharmazeutische Darreichungsformen.

Aufgrund der enormen Flexibilität und der niedrigen Viskosität sind bei der Verwendung als Überzugsmittel in der Regel keine zusätzlichen Weichmacher erforderlich.

Bei den überzogenen Darreichungsformen handelt es sich bevorzugt u.a. um Filmtabletten, Filmmikrotabletten, Dragees, überzogene Pastillen, Kapseln, Kristalle, Granulate oder Pellets.

Als pharmazeutische Wirkstoffe können neben Arzneistoffen auch Vitamine, Nahrungsergänzungsmittel oder dietätische Wirkstoffe eingesetzt werden.

Die Darreichungsformen weisen eine verzögerte Freisetzung des Wirkstoffs auf. Solche Formen werden auch als Formen mit retardierter Freisetzung bezeichnet. Retardiert bedeutet in diesem Zusammenhang, dass die Freisetzung über einen Zeitraum von 4 bis 36 Stunden erfolgt und dass die Freisetzung von 80 % des Wirkstoffs frühestens nach vier Stunden erfolgt ist. Damit unterscheiden sich Retardformen von schnellfreisetzenden Formen, bei denen 80 % des Wirkstoffs spätestens nach einer Stunde freigesetzt sind.

Die Formen setzen ph-unabhängig frei. Eine pH-unabhängige Freisetzung bedeutete, dass die Freisetzung in künstlichem Magensaft (0.1 n HCl; pH 1,2) sich von der Freisetzung in künstlichem Darmsaft (Phosphatpuffer; pH 6,8) nicht unterscheidet. D.h. die Differenz in der Freisetzung zu jedem Prüfzeitpunkt soll nicht mehr als 10% betragen.

Zur Anwendung für pharmazeutische Darreichungsformen können den Dispersionen zusätzlich übliche pharmazeutisch akzeptable Hilfsstoffe ausgewählt aus der Gruppe bestehend aus Antischaummitteln, Füllstoffen, Farbstoffen, Pigmenten, Antioxidantien, Konservierungsmitteln, Glanzverstärkern und Weichmachern in den hierfür üblichen Mengen zugegeben werden. Bei niedrig löslichen Wirkstoffen kann es sich empfehlen zur Steuerung der Freisetzung zusätzlich einen Porenbildner zuzusetzen. Als Porenbildner eignen sich beispielsweise Hydroxypropylmethylcellulose, Polyvinylalkohol-Polyethylenglykol-Pfropfcopolymere wie Kollicoat® IR, Polyvinylalkohole, Vinylpyrrolidon-Homo- und Copolymere, natürliche Polysaccharide, oder niedermolekulare wasserlösliche Stoffe wie Zucker, Zuckeralkohole, anorganische Salze. Solche Porenbildner können je nach Löslichkeit in Mengen von 1 bis 30, bevorzugt 2 bis 20 Gew.-%, bezogen auf Filmbildner.
Insgesamt kann der Anteil an Hilfsstoffen bis zu 50 Gew.-%, bezogen auf den Feststoffgehalt der Dispersion, betragen.

Ferner können auch wasserunlösliche aber wasserquellbare Stoffe in kleiner Korngröße wie mikrokristalline Cellulose, quervernetzte Natriumcarboxymethylcellulose oder - stärke und quervernetztes Polyvinylpyrrolidon eingesetzt werden.

Ein besonderer Vorteil der wässrigen Dispersionen liegt unter anderem in ihrer geringen Neigung zum Schäumen und damit verminderter Neigung zur Stippenbildung. Außerdem weisen die Dispersionen eine sehr gute Stabilität auf, was sich and er Scherstabilität und Sedimentationsstabilität ablesen lässt. Die erfindungsgemäß erhaltenen Überzüge weisen neben dem niedrigen Emulgatorgehalt weitere Vorzüge auf. So ist die Klebrigkeit der Filme gegenüber vergleichbaren kommerziellen Mitteln verringert. Zudem weisen die Überzüge hervorragende mechanische Eigenschaften auf.

In den nachfolgenden Beispielen wird die Herstellung der wässrigen Polymerdispersionen und deren Anwendung näher erläutert.

### Herstellung der Polymerisate

### Allgemeine Vorschrift

Das Polymerisationsgefäß wurde mit Stickstoff gespült. Das VE-Wasser (vollentsalztes Wasser)für die Vorlage wurde unter Rühren (120 Upm) in den Polymerisationskessel gegeben. Anschliessend wurde das Natriumlaurylsulfat (als 15 gew.-%ige wässrige Lösung) in die Vorlage gegeben, und mit VE-Wasser nachgespült. Die Vorlage wurde auf 80°C Innentemperatur geheizt. Beim Erreichen von 70°C Innentemperatur wurde der Zulauf 1 schnell zugegeben. Bei 75°C wurde der Zulauf 2 (7 gew.-%ige wässrige Natriumperoxodisulfatlösung) schnell zugegeben. Bei 80°C wurde mit den Zuläufen 3 (Emulsionszulauf) und 4 (2,5 gew.-%ige wässrige Natriumperoxodisulfatlösung) begonnen. Die Zuläufe 3 und 4 wurden über einen Zeitraum von 2 Stunden zugegeben. Nach Ende der Zuläufe 3 und 4 wurde zwei Stunden nachpolymerisiert. Nach Ende der Nachpolymerisation wurde das Polymerisationsgemisch auf 20°C gekühlt. Dann wurde der Zulauf 5 zugeben und 30 Minuten gemischt.

### Beispiel 1

Nach der allgemeinen Vorschrift wurde eine Dispersion mit einem Monomerverhältnis EA/MMA/MAS = 65,6/33,9/0,5 Gew.-% hergestellt. Der Emulgatoranteil betrug 0,3 Gew.%, bezogen auf das Monomerengewicht.

| Vorlage | |
|---|---|
| 413,0 g | VE-Wasser |
| 3,85 g | Natriumlaurylsulfat (15 gew.-%ige wässrige Lösung) |
| 5,10 g | VE-Wasser |
| | |

| Zulauf 01 | |
|---|---|
| 45,00 g | von Zulauf 3 |
| | |

| Zulauf 02 | |
|---|---|
| 6,35 g | Natriumperoxodisulfat (7 gew.-%ige wässrige Lösung) |
| | |

| Zulauf 03 | |
|---|---|
| 413,6 g | VE-Wasser |
| 5,0 g | Natriumlaurylsulfat (15% gew.-%ige wässrige Lösung) |
| 290,8 g | Ethylacrylat |
| 150,3 g | Methylmethacrylat |
| 2,2 g | Methacrylsäure |
| 7,6 g | VE- Wasser |
| | |

| Zulauf 04 | |
|---|---|
| 17,8 g | Natriumperoxodisulfat (2,5 gew.-%ige wässrige Lösung) |
| | |

| Zulauf 05 | |
|---|---|
| 125,2 g | VE- Wasser |
| 3,55 g | Natronlauge (10 gew.-%ige wässrige Lösung) |

Die bläulich weiße wässrige Dispersion wies einen Feststoffgehalt von 31 Gew.% auf.
Die mittlere Teilchengröße betrug 149 nm.

### Beispiel 2

Dispersion mit einem Monomerverhältnis EA/MMA/MAS = 66/33,9/ 0,1 Gew.-%. Der Emulgatoranteil betrug 0,3 Gew.-% bezogen auf Monomere.
Die Monomereinwaage betrug

| | |
|---|---|
| 292,0 g | Ethylacrylat |
| 150,9 g | Methylmethacrylat |
| 0,44 g | Methacrylsäure |

Die bläulich weiße wässrige Dispersion wies einen Feststoffgehalt von 31 Gew.-% auf.
Die mittlere Teilchengröße betrug 141 nm.

### Beispiel 3

Beispieldispersion mit einem Monomerverhältnis
EA/MMA/MAS = 65,8/34/0,2 Gew.-%. Der Emulgatoranteil betrug 0,3 Gew.% bezogen auf Monomere.
Die Monomereinwaage betrug

| | |
|---|---|
| 291,7 g | Ethylacrylat |
| 150,7 g | Methylmethacrylat |
| 0,88 g | Methacrylsäure |

Die bläulich weiße wässrige Dispersion wies einen Feststoffgehalt von 31 Gew.-% auf.
Die mittlere Teilchengröße
betrug 154 nm.

### Beispiel 4

Analog lässt sich eine Dispersion mit einem Monomerverhältnis EA/MMA/MAS = 64/32/4 Gew.-% bei einem Emulgatoranteil von 0,3 Gew.-%, bezogen auf das Monomerengewicht, herstellen.

| Monomereinwaage: | |
|---|---|
| 284,0 g | Ethylacrylat |
| 142,0 g | Methylmethacrylat |
| 18,0 g | Methacrylsäure |

Die Einstellung des pH-Wertes auf pH 7 erfolgt mit 3 gew.-%iger wässriger Natronlauge.

### Beispiel 5

Analog Beispiel 1 wird ein Polymerisat mit einem Monomerverhältnis EA/MMA/MAS = 66/33,7/0,3 Gew.-% bei einem Emulgatoranteil von 0,3 Gew.-%, bezogen auf das Monomerengewicht, hergestellt.

| Monomereinwaage: | |
|---|---|
| 292,8 g | Ethylacrylat |
| 144,5 g | Methylmethacrylat |
| 1,32 g | Methacrylsäure |

Die Einstellung des pH-Wertes auf pH 7 erfolgt mit 3 gew.-%iger wässriger Natronlauge.

### Anwendungsbeispiele

Sofern nicht anders angegeben beziehen sich %-Angaben auf Gew.-%.

### Beispiel 6

1,0kg Propranolol-HCl Pellets (20 Gew.-% Propranolol-HCl, 51,7 % mikrokristalline Cellulose, 25,8 % Lactose, 2,5 % Vinylpyrrolidon-Vinylacetat-(6:4)-Copolymer) mit einer Korngröße 0,8 - 1,5 mm hergestellt durch Feuchtextrusion und anschließende Sphäronisation wurden in einem Wirbelschichtcoater Glatt GPC G3 mit folgender Coatingrezeptur gecoatet:

| | |
|---|---|
| Ethylacrylat-Methylmethacrylat)Copolymer-Dispersion mit 0,1% Methacrylsäure gemäß Beispiel 2 | 300,0 g |
| Talkum | 40,0 g |
| Simethicon | 0,5 g |
| Wasser | 190,0 g |

Zur Herstellung der Coatingrezeptur wurden Simethicon und Talkum in 190,0 g Wasser eingerührt und mittels eines Ultra-turrax bei 8000 rpm 10 min dispergiert. Diese Zubereitung wurde langsam in die Dispersion eingerührt. Nach weiteren 30 min Rühren war die Coatingsuspension gebrauchsfertig.

Die Coatingsuspension wurde bei einer Zulufttemperatur von 38°C und einer Sprührate von 13 g/min mittels Wurstereinsatz auf die wirbelnden Pellets appliziert. Nach Beendigung des Sprühprozesses wurde noch 5 Minuten bei 38°C Zulufttemperatur nachgetrocknet. Die Freisetzung des Wirkstoffes wurde in einer Paddleapparatur nach USP 23 (Fa. Pharmatest PTW) bestimmt, wobei sowohl in 0,1 N HCl als auch in Phosphatpuffer pH 6,8 freigesetzt wurde.

Folgende Freisetzungswerte wurden erhalten:

| Freisetzung nach | 0,1 N HCl (pH 1,2) | Phosphatpuffer pH 6,8 |
|---|---|---|
| 1h | 1,4 % | 1,6 % |
| 2h | 5,6 % | 5,9 % |
| 4h | 27,4 % | 28,5 % |
| 8h | 65,3 % | 65.1 % |
| 12h | 84,8 % | 84,1 % |
| 16h | 99,8 % | 100,3 % |

Die Freisetzung war vollkommen pH-unabhängig.

### Beispiel 7

1,0 kg Coffein Pellets (20 % Coffein, 55,0 % mikrokristalline Cellulose, 22,5 % Lactose, 2,5 % Vinylpyrrolidon-Vinylacetat-(6:4)-Copolymer) mit einer Korngröße 0,7 - 1,6 mm hergestellt durch Feuchtextrusion und anschließende Sphäronisation wurden in einem Wirbelschichtcoater Glatt GPC G3 mit folgender Coatingrezeptur gecoatet:

| | |
|---|---|
| Copolymer-Dispersion mit 0,2 % Methacrylsäure gemäß Beispiel 3 | 400,0 g |
| Hydroxypropylmethylcellulose 3 mPas | 12,0 g |
| Talkum | 40,0 g |
| Simethicon | 0,5 g |
| Wasser | 340,0 g |

Zur Herstellung der Coatingrezeptur wurden Hydroxypropylmethylcellulose, Simethicon und Talkum in 340,0 g Wasser eingerührt und mittels eines Ultra-turrax bei 8000 rpm 15 min dispergiert. Diese Zubereitung wurde langsam in die Ethylacrylat-Methylmethacrylat-Copolymer-Dispersion gemäß Beispiel 3 eingerührt. Nach weiteren 30 min Rühren war die Coatingsuspension gebrauchsfertig.

Die Coatingsuspension wurde bei einer Zulufttemperatur von 42°C und einer Sprührate von 16 g/min mittels Wurstereinsatz auf die wirbelnden Pellets appliziert. Nach Beendigung des Sprühprozesses wurde noch 5 Minuten bei 42°C Zulufttemperatur nachgetrocknet. Die Freisetzung des Wirkstoffes wurde in einer Paddleapparatur nach USP 23 (Fa. Pharmatest PTW) bestimmt, wobei sowohl in 0,1 N HCl und als auch in Phosphatpuffer pH 6,8 freigesetzt wurde.

Folgende Freisetzungswerte wurden erhalten:

| Freisetzung nach | 0,1 N HCl (pH 1,2) | Phosphatpuffer pH 6,8 |
|---|---|---|
| 1 h | 1,0 % | 0,8 % |
| 2 h | 4,5 % | 4,3 % |
| 4h | 11,7% | 11,9% |
| 8 h | 31,3 % | 31,1 % |
| 12 h | 53,6 % | 52,9 % |
| 16 h | 72,8 % | 72,1 % |
| 20 h | 87,2 % | 86,3 % |
| 24 h | 99,4 % | 98,1 % |

Die Freisetzung war vollkommen pH-unabhängig.

### Beispiel 8

2,0 kg Theophyllin Pellets (60 % Theophyllin, 37,5 % mikrokristalline Cellulose, 2,5 % Vinylpyrrolidon-Vinylacetat-(6:4)-Copolymer) mit einer Korngröße 0,7 -1,4 mm hergestellt durch Feuchtextrusion und anschließende Sphäronisation wurden in einem Wirbelschichtcoater Glatt GPC G3 mit folgender Coatingrezeptur gecoatet:

| | |
|---|---|
| Dispersion gemäß Beispiel 3 | 400,0 g |
| mikrokristalline Cellulose | 10,0 |
| Titandioxid | 10,0g |
| Kaolin | 10,0 g |
| Simethicon | 1,0 g |
| Wasser | 300,0 g |

Zur Herstellung der Coatingrezeptur wurden Simethicon, mikrokristalline Cellulose, Titandioxid und Kaolin in 300,0 g Wasser eingerührt und mittels eines Ultra-turrax bei 8000 rpm 10 min dispergiert. Diese Zubereitung wurde langsam in die Copolymer-Dispersion 30 % eingerührt. Nach weiteren 30 min Rühren war die Coatingsuspension gebrauchsfertig.

Die Coatingsuspension wurde bei einer Zulufttemperatur von 41°C und einer Sprührate von 18 g/min mittels Wurstereinsatz auf die wirbelnden Pellets appliziert. Nach Beendigung des Sprühprozesses wurde noch 5 Minuten bei 41 °C Zulufttemperatur nachgetrocknet. Die Freisetzung des Wirkstoffes wurde in einer Paddleapparatur nach USP 23 (Fa. Pharmatest PTW) bestimmt, wobei 2 h in 0,1 N-HCl und anschließend in Phosphatpuffer pH 6,8 freigesetzt wurde.

Parallel wurde ein Versuch mit der gleichen Rezeptur, aber unter Verwendung von Kollicoat EMM 30 D (Copolymer aus Ethylacrylat/Methylmethacrylat 2:1, enthaltend 1,5 Gew.-% ethoxyliertes Nonylphenol, bezogen auf die 30%ige wässrige Dispersion), durchgeführt und ebenfalls die Freisetzung bestimmt.

Folgende Freisetzungswerte wurden erhalten:

| Freisetzung nach | Dispersion gemäß Beispiel 3 | Kollicoat EMM 30 D |
|---|---|---|
| 1 h | 0,8 % | 1,6 % |
| 2 h | 3,6 % | 5,9 % |
| 4h | 11,4 % | 17,5 % |
| 8 h | 25,9 % | 38.2 % |
| 12 h | 48,6 % | 61,2 % |
| 16 h | 64,2 % | 81,3 % |
| 20 h | 81,5 % | 96,6 % |
| 24 h | 96,5 % | 101,1 % |

Standardabweichung der Freisetzungswerte aus 12 individuellen Freisetzungen:

| | |
|---|---|
| Dispersion gemäß Beispiel 3: | 3,1 % |
| Kollicoat EMM 30 D: | 5,9 % |

Die Freisetzung des Produktes nach Beispiel 3 war deutlich langsamer als die mit Kollicoat EMM 30 D (mit ethoxyliertem Nonylphenol).
Darüber hinaus war die Reproduzierbarkeit der Freisetzung bei der Dispersion nach Beispiel 3 deutlich besser.

### Beispiel 9

Zunächst wurden Ambroxol-HCl Pellets (10 % Ambroxol-HCl, 80,0 % Saccharose, 10,0 % Vinylpyrrolidon-Vinylacetat-(6:4)-Copolymer) durch Drug Layering von Saccharosepellets einer Korngröße 0,7-0,9 mm hergestellt. Dazu wurde eine Lösung von 7,5 % Vinylpyrrolidon-Vinylacetat-(6:4)-Copolymer) und 7,5 % Ambroxol-HCl in Wasser auf die Zuckerpellets in einem Wirbelschichtcoater aufgesprüht bis die Pellets einen Wirkstoffgehalt von 10 % aufwiesen. 1,0 kg dieser Wirkstoffpellets wurden anschließend in einem Wirbelschichtcoater Aeromatic Strea 1 mit folgender Coatingrezeptur gecoatet:

| | |
|---|---|
| Dispersion gemäß Beispiel 1 | 370,0 g |
| Eisenoxid | 8,0 g |
| Titandioxid | 10,0 g |
| Polyvinylalkohol-Polyethylenglykol Pfropfcopolymer (Kollicoat IR | 6,0 |
| Simethicon | 0,5 g |
| Wasser | 300,0 g |

Zur Herstellung der Coatingrezeptur wurden Eisenoxid, Simethicon, Polyvinylalkohol-Polyethylenglykol Pfropfcopolymer und Titandioxid in 300,0 g Wasser eingerührt und mittels eines Ultra-turrax bei 8000 rpm 8 min dispergiert. Diese Zubereitung wurde langsam in die Dispersion gemäß Beispiel 1 eingerührt. Nach weiteren 30 min Rühren war die Coatingsuspension gebrauchsfertig.

Die Coatingsuspension wurde bei einer Zulufttemperatur von 40°C und einer Sprührate von 12 g/min nach dem Topsprayverfahren auf die wirbelnden Pellets appliziert. Nach Beendigung des Sprühprozesses wurde noch 5 Minuten bei 40°C Zulufttemperatur nachgetrocknet.
Die Freisetzung des Wirkstoffes wurde in einer Paddleapparatur nach USP 23 (Fa. Pharmatest PTW) bestimmt, wobei in Phosphatpuffer pH 7,4 freigesetzt wurde.

Folgende Freisetzungswerte wurden erhalten:

| Freisetzung nach | freigesetzte Menge |
|---|---|
| 1 h | 3,6 % |
| 2 h | 8.3 % |
| 4 h | 20,5 % |
| 8 h | 42,6 % |
| 12 h | 61,6 % |
| 16 h | 78,2 % |
| 20 h | 95,0 % |

### Beispiel 10

### Sedimentationsstabilität

Bestimmt in einer Zentrifuge bei 4000 rpm entsprechend 2750 g (2750 fache Erdbeschleunigung) und einem Füllstand des Behältnisses von 80 mm. Nach dem vorsichtigen Ausleeren des Behältnisses wurde der verbliebene Rückstand in mm abgelesen.

| Dispersion gemäß | Höhe Bodensatz [mm] | |
|---|---|---|
| | nach 90 min | nach 150 min |
| Kollicoat EMM 30 D | 7 | 9 |
| Beispiel 2 (0,1% Methacrylsäure) | 6 | 7 |
| Beispiel 3 (0,2 % Methacrylsäure) | 5 | 7 |
| Beispiel 1 (0,5 % Methacrylsäure) | 5 | 6 |

Die Dispersionen nach Beispiel 1-3 weisen eine bessere Sedimentationsstabilität auf als das Vergleichsprodukt Kollicoat EMM 30 D mit ethoxyliertem Nonylphenol. Die Dispersionen nach Beispiel 1-3 sind lagerstabiler.

### Beispiel 11

### Scherstabilität

Bestimmt wurde die Scherstabilität mit einem Noppenrührer, der 8 Rührstifte und 4 Durchflußbohrungen aufweist. Dieser Noppenrührer taucht in die Dispersion ein und dreht 15 min bei 2000 rpm. Anschließend wird die Dispersion über ein 125 µm-Sieb gegeben, nachgewaschen und der Rückstand nach Trocknung bei 105°C bestimmt.

| Dispersion | Koagulat [%] |
|---|---|
| Kollicoat EMM 30 D | 4,6 |
| Beispiel 2 (0,1 % Methacrylsäure) | 0 |
| Beispiel 3 (0,2 % Methacrylsäure) | 0 |
| Beispiel 1 (0,5 % Methacrylsäure) | 0 |

Die Dispersionen nach Beispiel 1-3 sind gegenüber Scherbeanspruchungen wesentlich stabiler als das Vergleichsprodukt Kollicoat EMM 30D mit ethoxyliertem Nonylphenol. Koagulationen während der Anwendung beim Überziehen von Darreichungsformen treten daher nicht auf.

### Beispiel 12

Klebrigkeit von Ethylacrylat-Methylmethacrylat(2:1)Copolymer-Filmen bestimmt nach der Methode von Hoessel

Die Methode von Hoessel ist beschrieben in Cosmetics and Toiletries, 111 (8),73-77 (1996). Dabei wird ein Carbonstempel auf den Film gedrückt und die Schwärzung des Filmes ist ein Maß für die Klebrigkeit des Filmes.

| Bewertungsmassstab: | |
|---|---|
| Stark klebrig | 5 |
| Nicht klebrig | 0 |

| Dispersion | Klebrigkeit bei 20°C/80 % rel.Feuchte. |
|---|---|
| Kollicoat EMM 30 D | 3,25 |
| Beispiel 2 (0,1 % Methacrylsäure) | 2,75 |
| Beispiel 3 (0,2 % Methacrylsäure) | 2,5 |
| Beispiel 1 (0,5 % Methacrylsäure) | 2,25 |

Die Klebrigkeit der Filme nach Beispiel 1-3 ist deutlich niedriger als die des Vergleichsproduktes Kollicoat EMM 30 D mit ethoxyliertem Nonylphenol.

### Beispiel 13

### Mechanische Eigenschaften von Ethylacrylat-Methylmethacrylat(2:1)Copolymer-Filmen

E-Modul und Reißdehnung wurden mit einem Texture Analyzer TA-XT2 HiR der Firma Stable Microsystems nach Konditionierung der Filme bei 23°C/54 % relativer Feuchte bestimmt.

| Dispersion | E-Modul [MPa] | Reißdehnung [%] |
|---|---|---|
| Kollicoat EMM 30 D | 16 | >450 |
| Beispiel 2 (0,1 % Methacrylsäure) | 45 | >450 |
| Beispiel 3 (0,2 % Methacrylsäure) | 48 | >450 |
| Beispiel 1 (0,5 % Methacrylsäure) | 51 | >450 |

Die Filme nach Beispiel 1-3 sind so flexibel wie die des Vergleichsproduktes Kollicoat EMM 30 D mit ethoxyliertem Nonylphenol, aber deutlich härter, erkennbar am höheren E-Modul. Härtere Überzüge sind in der Anwendung von Vorteil, da sie durch mechanische Beanspruchung nicht so schnell beschädigt werden.

## Patentansprüche

1. Verwendung von wässrigen Polymerdispersionen als Binde- und Überzugsmittel für pharmazeutische Darreichungsformen, wobei die wässrigen Polymerdispersionen durch radikalische Polymerisation in einem wässrigen System in Gegenwart eines Polymerisationsinitiators von
i) 60 bis 99.95 Gew.-% mindestens eines Monomers ausgewählt aus der Gruppe bestehend aus Alkylacrylaten, Alkylmethacrylaten und Vinylestern von C₁-C₂₄-Carbonsäuren,
ii) 0,05 bis 5 Gew.-% einer monolefinisch ungesättigten C3-C8-Carbonsäure
iii) 0 bis 35 Gew.-% weiterer radikalisch polymerisierbarer Monomere,
wobei die Summe von i), ii), und iii) gleich 100 Gew.-% ist, in Gegenwart von 0,001 bis 1,0 Gew.-%, bezogen auf das Gesamtgewicht der Monomeren, eines anionischen Emulgators, erhalten werden.

2. Verwendung nach Anspruch 1 , wobei Polymerisate aus
i) 95 bis 99.95 Gew.-% mindestens eines Monomers ausgewählt aus der Gruppe bestehend aus Alkylacrylaten, Alkylmethacrylaten und Vinylestern von C₁-C₂₄-Carbonsäuren,
ii) 0,05 bis 5 Gew.-% einer monolefinisch ungesättigten C3-C8-Carbonsäure,
wobei die Summe von i) und ii) gleich hundert ist, eingesetzt werden.

3. Verwerdung nach Anspruch 1 oder 2, wobei Polymerisate aus
i) 95 bis 99.95 Gew.-% mindestens eines Monomers ausgewählt aus der Gruppe bestehend aus Alkylacrylaten und Alkylmethacrylaten,
ii) 0,05 bis 5 Gew.-% einer monolefinisch ungesättigten C3-C8-Carbonsäure,
wobei die Summe von i) und ii) gleich hundert ist, eingesetzt werden..

4. Verwendung nach einem der Anspruche 1 bis 3, wobei Polymerisate aus
i) 95 bis 99.95 Gew.-% mindestens eines Monomers ausgewählt aus der Gruppe bestehend aus Alkylacrylaten und Alkylmethacrylaten , wobei das Gewichtsverhältnis von Alkylacrylaten zu Alkylmethacrylaten 9:1 bis 1:3 beträgt, und
ii) 0,05 bis 5 Gew.-% einer monolefinisch ungesättigten C₃-C₈-Carbonsäure,
wobei die Summe von i) und ii) gleich hundert ist, eingesetzt werden

5. Verwendung nach einem der Ansprüche 1 bis 4, wobei das Gewichtsverhältnis von Alkylacrylaten zu Alkylmethacrylaten 3:1 bis 1:1 beträgt.

6. Verwerdung nach einem der Ansprüche 1 bis 5, wobei das Gewichtsverhältnis von Alkylacrylaten zu Alkylmethacrylaten 2,5:1 bis 1,5:1 beträgt.

7. Verwendung nach einem der Ansprüche 1 bis 6, wobei als Monomere i) Ethylacrylat und Methylmethacrylat eingesetzt werden.

8. Verwendung nach einem der Ansprüche 1 bis 7, wobei als Monomer ii) Methacrylsäure eingesetzt wird.

9. Verwendung nach einem der Ansprüche 1 bis 8, wobei als anionischer Emulgator mindestens eine Verbindung ausgewählt aus der Gruppe bestehend aus Ammoniumsalzen oder Alkalisalzen von C8-C30-Fettsäuren, Alkylsulfaten, Alkylsulfonaten oder Alkylarylsulfonaten, wobei die Alkylreste 8 bis 30 C-Atome aufweisen, Sulfobernsteinsäure-mono- und -di-estern und sulfatierten Fettalkoholethoxylaten, eingesetzt wird.

10. Verwendung nach einem der Ansprüche 1 bis 9 als Überzugsmittel für pharmazeutische Darreichungsformen.

11. Verwendung nach einem der Ansprüche 1 bis 10, wobei die Dispersionen als zusätzliche Hilfsmittel pharmazeutisch akzeptable Hilfsstoffe ausgewählt aus der Gruppe bestehend aus Antischaummittteln, Füllstoffen, Farbstoffen, Pigmenten, Anitoxidantien, Konservierungsmitteln, Porenbildnern, Glanzverstärkern und Weichmachern enthalten.

## Claims

1. The use of aqueous polymer dispersions as binder and coating agents for pharmaceutical dosage forms, where the aqueous polymer dipersions are obtained by free radical polymerization in an aqueous system in the presence of a polymerization initiator of
i) 60 to 99.95% by weight of at least one monomer selected from the group consisting of alkyl acrylates, alkyl methacrylates and vinyl esters of C₁-C₂₄ carboxylic acids,
ii) 0.05 to 5% by weight of a monoolefinically unsaturated C₃-C₃ carboxylic acid
iii) 0 to 35% by weight of further free-radically polymerizale monomers,
where the total of i), ii), and iii) equals 100% by weight, in the presence of 0.001 to 1.0% by weight, based on the total weight of the monomers, of an anionic emulsifier.

2. The use according to claim 1, where polymers of
i) 95 to 99.95% by weight of at least one monomer selected from the group consisting of alkyl acrylates, alkyl methacrylates and vinyl esters of C₁-C₂₄ carboxylic acids,
ii) 0.05 to 5% by weight of a monoolefinically unsaturated C₃-C₈ carboxylic acid,
where the total of i) and ii) equals one hundred, are employed.

3. The use according to claim 1 or 2, where polymers of
i) 95 to 99.95% by weight of at least one monomer selected from the group consisting of alkyl acrylates and alkyl methacrylates,
ii) 0.05 to 5% by weight of a monoolefinically unsaturated C₃-C₈ carboxylic acid,
where the total of i) and ii) equals one hundred, are employed.

4. The use according to any of claims 1 to 3, where polymers of
i) 95 to 99.95% by weight of at least one monomer selected from the group consisting of alkyl acrylates and alkyl methacrylates, where the ratio of alkyl acrylates to alkyl methacrylates is from 9:1 to 1:3 by weight, and
ii) 0.05 to 5% by weight of a monoolefinically unsaturated C₃-C₈ carboxylic acid,
where the total of i) and ii) equals one hundred, are employed.

5. The use according to any of claims 1 to 4, where the ratio of alkyl acrylates to alkyl methacrylates is from 3:1 to 1:1 by weight.

6. The use according to any of claims 1 to 5, where the ratio of alkyl acrylates to alkyl methacrylates is from 2.5:1 to 1.5:1 by weight.

7. The use according to any of claims 1 to 6, where ethyl acrylate and methyl methacrylate are employed as monomers i).

8. The use according to any of claims 1 to 7, where methacrylic acid is employed as monomer ii)

9. The use according to any of claims 1 to 8, where, as anionic emulsifier, at least one compound selected from the group consisting of ammonium salts or alkali metal salts of C₈-C₃₀ fatty acids, alkyl sulfate, alkylsulfonates or alkylarylsulfonates, where the alkyl radicals have 3 to 30 C atoms, sulfosuccinic monoesters and diesters and sulfated fatty alcohol ethoxylates, is employed.

10. The use according to any of claims 1 to 9 as coating agent for pharmaceutical dosage forms.

11. The use according to any of claims 1 to 10, where the dispersions comprise as additional aids pharmaceutically acceptable excipients selected from the group consisting of antifoams, fillers, colors, pigments, antioxidants, preservatives, pore formers, gloss improvers and plasticizers.

## Revendications

1. Utilisation de dispersions polymères aqueuses comme agents liants et agents de revêtement pour des formes galéniques pharmaceutiques, les dispersions polymères aqueuses étant obtenues par une polymérization radicalaire dans un système aqueux en présence d'un initiateur de polymérisation de :
i) 60 à 99,95% en poids d'au moins un monomère choisi parmi le groupe comprenant des alkylacrylates, des alkylméthacrylates et des esters vinyliques d'acides carboxyliques en C₁-C₂₄,
ii) 0,05 à 5 % en poids d'un acide carboxylique en C₃-C₈ monooléfiniquement insaturé,
iii) 0 à 35% en poids d'autres monomères radicalement polymérisables,
la somme de i) , ii), et iii) étant égale à 100 % en poids, en présence de 0,001 à 1,0% en poids, par rapport au poids total des nonomères, d'un émulsifiant anionique.

2. Utilisation selon la revendication 1, des polymères étant utilisés qui se composent de :
i) 95 à 99,95 % en poids d'au moins un monomère choisi parmi le groupe comprenant des alkylacrylates, des alkylméthacrylates et des esters vinyliques d'acides carboxyliques en C₁-C₂₄.
ii) 0,05 à 5 % en poids d'un acide carboxylique en C₃-C₈ monooléfiniquement insaturé,
la somme de i) et ii) étant égale à cent.

3. Utilisation selon la revendication 1 ou 2, des polymères étant utilisés qui se composent de :
i) 95 à 99,95 % en poids d'au moins un monomère choisi parmi le groupe comprenant des alkylacrylates et des alkylméthacrylates,
ii) 0,05 à 5 % en poids d'un acide carboxylique en C₃-C₈ monooléfiniquement insaturé,
la somme de i) et ii) étant égale à cent.

4. Utilisation selon l'une quelconque des revendications 1 à 3, des polymères étant utilisés qui se composent de :
i) 95 à 99,95 % en poids d'au moins un monomère choisi parmi le groupe comprenant des alkylacrylates et des alkylméthacrylates, le rapport en poids entre les alkylacrylates et les alkylméthacrylates étant de 9:1 à 1:3, et
ii) 0,05 à 5 % en poids d'un acide carboxylique en C₃-C₈ monooléfiniquement insaturé,
la somme de i) et ii) étant égale à cent.

5. Utilisation selon l'une quelconque des revendications 1 à 4, le rapport en poids entre les alkylacrylates et les alkylméthacrylates étant de 3:1 à 1:1.

6. Utilisation selon l'une quelconque des revendications 1 à 5, le rapport en poids entre les alkylacrylates et les alkylméthacrylates étant de 2, 5:1 à 1, 5:1.

7. Utilisation selon l'une quelconque des revendications 1 à 6, un éthylacrylate et un méthylméthacrylate étant utilisés comme monomères

8. Utilisation selon l'une quelconque des revendications 1 à 7, de l'acide méthacrylique étant utilisé comme monomère ii).

9. Utilisation selon l'une quelconque des revendications 1 à 8, au moins un composé, qui est utilisé comme émulsifiant anionique, étant choisi parmi le groupe se composant de sels d'ammonium ou de sels alcalins d'acides gras en C₈-C₃₀, d'alkylsulfates, d'alkylsulfonates ou d'alkylarylsulfonates, les radicaux alkyle comportant de 8 à 30 atomes de C, de monoesters et diesters de l'acide sulfosuccinique et d'éthoxylates d'alcool gras sulfatés.

10. Utilisation selon l'une quelconque des revendications 1 à 9 en tant qu'agent de revêtement pour des formes galéniques pharmaceutiques.

11. Utilisation selon l'une quelconque des revendications 1 à 10, les dispersions contenant, en tant que moyens auxiliaires supplémentaires, des adjuvants pharmaceutiquement acceptables choisis parmi le groupe comportant des agents anti-mousse, des matières de charge, des colorants, des pigments, des anti-oxydants, des agents de conservation, des agents porogènes, des amplificateurs de brillance et des plastifiants.
